# EUROPEAN PATENT APPLICATION

(11) **EP 4 074 240 A1**
(43) Date of publication of application: **19.10.2022**
(21) Application number: 21168178.8
(22) Date of filing: 13.04.2021
(51) Int. Cl.: A61B 1/00

(54) **ENDOSCOPE IMAGE RECORDING UNIT**

(71) Applicant: Ambu A/S, 2750 Ballerup (DK)
(72) Inventor: NIELSEN, Brian, 4700 Næstved (DK)
(74) Representative: AWA Denmark A/S

(57) **Abstract**

Disclosed is an image recording unit for displaying endoscope images. The display unit comprising a processing unit, the processing unit being operationally connectable to an image capturing device, a display and one or more memory units. The processing unit is configured to: continuously receive images captured by the image capturing device; continuously update a temporary image buffer, the temporary image buffer storing a stream of images received within a first period back in time; in response to a received user input, storing permanently a stream of images, wherein the permanently stored stream of images includes a subset of the stream of images stored in the temporary image buffer, and wherein the subset is selected dependent on the user input so that a user may select the point back in time where the user wants the permanently stored stream of images to start.

## Description

### Field

The present disclosure relates to an image recording unit, an endoscopic system, a method of recording images and a computer program product.

### Background

Endoscopes are widely used in hospitals for visually examining body cavities and obtaining samples of tissue identified as potentially pathological. An endoscope typically comprises an image capturing unit arranged at the distal end of the endoscope either looking forward or to the side. An endoscope is further typically provided with a working channel allowing a medical device such as a gripping device, a suction device, or a catheter to be introduced.

An important part of the work for a medical professional is to document the result of an endoscopic procedure. As an example, a medical professional may need to document the discovery of a pathological condition. Additionally, a medical professional may need to document that a particular part of body has been examined.

This is typically done by saving still images or videos from different parts of the examination. In theory a video of an entire endoscopic examination could be saved. However, such an approach would result in very large datasets. Furthermore, it may be difficult afterwards to find the important parts of the video.

Most endoscopic system are therefore provided with a button either on the endoscope handle or on a display allowing a medical professional to start recording a video.

However, if the medical professional does not timely push the button a clinical important moment may be missed. This may be especially problematic if the clinical important moment included a special movement of an organ, as such a movement may not happen again for a long period of time. Furthermore, even if the medical professional just forgot to document that a particular part of the body has been examined, it may be highly problematic as the medical professional then would need to re-examine the part of the body resulting in a waste of time and unnecessary discomfort for the patient.

Thus it remains a problem to provide an improved device / system for documenting endoscopic procedures.

### Summary

According to a first aspect, the present disclosure relates to an image recording unit for recording endoscope images captured by an image capturing device of an endoscope, the image recording unit comprising a processing unit, the processing unit being operationally connectable to the image capturing device, a display, and one or more memory units, wherein the processing unit is configured to:
continuously receive images captured by the image capturing device of the endoscope;
continuously update a temporary image buffer stored in the one or more memory units with the received images, the temporary image buffer storing a stream of images received within a first period back in time;
in response to a received user input, storing permanently a stream of images recorded by the image capturing device in the one or more memory units, wherein the permanently stored stream of images includes a subset of the stream of images stored in the temporary image buffer, and wherein the subset is selected dependent on the user input so that a user may select the point back in time where the user wants the permanently stored stream of images to start.

Consequently, by allowing the user to start recording video from a point back in time, the risk that the user misses an important clinical moment may be reduced. Furthermore, by allowing the user to select a particular point back in time, only the clinical important moment may be captured and not a long period of time before the clinical important moment occurred. This may reduce the memory size of the permanently stored stream of images and further make it easier afterwards to find the clinical important moment.

The processing unit of the image processing device may be any processing unit, such as a central processing unit (CPU), a graphics processing unit (GPU), a microcontroller unit (MCU), a field-programmable gate array (FPGA), or any combination thereof. The processing unit may comprise one or more physical processors and/or may be combined by a plurality of individual processing units.

The image recording unit may comprise the display, i.e. the image recording unit may be integrated in a display unit. Alternatively / additionally, the image recording unit may be operationally connectable to a separate display unit.

The temporary image buffer may be updated each time the processing unit receives an image from the image capturing unit e.g. if the processing unit receives images with a frequency of 120 frames per second (FPS) then the stream of images stored in the temporary image buffer may also have a frequency of 120 FPS. However, the processing unit may also temporarily down-sample the images received e.g. if the processing unit receives images with a frequency of 120 frames per second (FPS) the stream of images stored in the temporary image buffer may have a frequency of 60 FPS.

The step of updating the temporary image buffer may comprise storing a new image. Additionally, the step of updating the temporary image buffer may comprise deleting the oldest image stored.

The temporary image buffer may be automatically deleted after an endoscopic procedure has been finished.

The length of the first period back in time may be a factory parameter setting that the user cannot change. Alternatively, it may be a parameter setting that the user may select typically before an endoscopic procedure is initiated e.g. as part of a setup procedure. The length of the first period back in time may be limited to only capture a part of a typical endoscopic procedure. Alternatively, the length of the first period back in time may be set so that it captures substantial all of a typical endoscopic procedure.

The user input may be received directly via the image recording unit e.g. the user input may be received via one or more buttons on the image recording unit. Alternatively, it may be received via another unit communicatively coupled to the image recording unit e.g. an endoscope handle may comprise on or more buttons that may be used by the user to provide the user input.

The stream of images permanently stored are not automatically deleted during or after a medical procedure. However, the user may have the option to manually delete the stream of images permanently stored.

The stream of images permanently stored may be stored with a FPS corresponding the FPS of the temporary image buffer.

In some embodiments the image recording unit comprises the one or more memory units.

In some embodiments the one or more memory units are part of another unit e.g. a server communicatively coupled to the processing unit.

In some embodiments the length of the first period is at least 8 second, at least 10 seconds, at least 30 seconds, or at least 1 minute, and preferably max 30 minutes.

Consequently, by having a temporary image buffer of a considerable length the user may be allowed to go back in time a significant amount. Furthermore, by not having the entire temporary image buffer stored each time a stream of images is permanently stored, the total memory usage of the image recording unit is not significantly increased by having a large temporary image buffer.

In some embodiments the user may select between at least three points back in time.

Consequently, the user may be provided with the flexibility to start a video at a desired point in time. This may secure that the video does not contain unnecessary content which only will result in an increase of memory usage and further make it more difficult afterwards to find the clinical important information.

The user may be provided with the opportunity to select any particular frame of the temporary image buffer. Alternative, the user may be able to select between a number of predetermined points in time e.g. 2 seconds, 5 seconds, 10 seconds or 30 seconds.

In some embodiments the display unit is configured to continuously display live images captured by the image capturing device of the endoscope.

In some embodiments the user input comprises a first user input and a second user input, the second user input being received after the first user input, wherein the processing unit in response to the first user input is configured to start storing the images received from the image capturing device forming a first stream of images, and wherein the processing unit in response to the second user input is configured to select the subset of the stream of images stored in the temporary image buffer, and wherein the stream of images permanently stored comprises the subset and the first stream of images.

Consequently, the user may have the opportunity to use more time to select the particular point back in time e.g. after the recording has been initated. This may be especially important if the temporary image buffer has a limited size.

The first user input and the second user input may be received from the same input unit e.g. the first user input and the second user input may be received from an input unit of the image recording unit or the handle of the endoscope. Alternatively, the first user input may be received from a first input unit and the second user input may be received from a second input unit e.g. the first user input may be received from an input unit of the endoscope handle and the second user input may be received from an input unit of the image recording unit.

The processing unit may also be configured to stop updating the temporary image buffer in response to the first user input.

This may be especially beneficial if the step of updating the temporary image buffer comprise deleting the oldest image stored as it may provide the user with more time to provide the second user input.

In some embodiments the processing unit in response to the first user input is configured to control the display to display live images recorded by the image capturing device of the endoscope in a first display zone of the display, and further images of the temporary image buffer in a second display zone of the display.

Consequently the user may both still be able to follow what goes on live in the body of the patient and be provided with visual feedback for selecting the point back in time.

In some embodiments the processing unit is configured to after having received the first user input replay the stream of images stored in the temporary image buffer in the second display zone preferably in a reverse direction, and wherein the processing unit further is configured to in response to the second user input select the subset of the stream of images stored in the temporary image buffer based on the image of the temporary image buffer shown in the second display zone at the time when the second user input is received.

In some embodiments the user input further comprises a third user input, the third user input being received after the first user input but before the second user input, the display is a touch display, and wherein the processing unit in response to the first user input is further configured to control the display to display a scrubber bar, and wherein the third user input is a selection and / or movement along the scrubber bar, the replay of the stream of images stored in the temporary image buffer is done in response to the selection and / or movement of the scrubber bar, and wherein the second user input is a release of the scrubber bar.

Consequently, a simple an intuitive way of allowing the user to selected the point back in time is provided.

In some embodiments the endoscope comprises one or more input elements, the image recording unit is operationally connectable to one or more input elements, and wherein the first user input and/or the second user input are received via the one or more input element of the endoscope.

Consequently, more parts of the system may be controlled via the endoscope which may enable the doctor to work more effectively.

In some embodiments the image recording unit is configured to provide power to an image capturing device of an endoscope.

According to a second aspect, the present disclosure relates to an endoscopic system comprising an image recording unit as disclosed in relation to the first aspect of the disclosure and an endoscope comprising an image capturing device.

According to a third aspect, the present disclosure relates to a method of recording images captured by an image capturing device of an endoscope, the method comprises the steps of:
continuously receive images captured by the image capturing device of the endoscope;
continuously update a temporary image buffer stored in one or more memory units with the received images, the temporary image buffer storing a stream of images received within a first period back in time;
in response to a received user input, storing permanently a stream of images recorded by the image capturing device in the one or more memory units, wherein the permanently stored stream of images includes a subset of the stream of images stored in the temporary image buffer, and wherein the subset is selected dependent on the user input so that a user may select the point back in time where the user wants the permanently stored stream of images to start.

According to a fourth aspect, the present disclosure relates to a computer program product comprising program code means adapted to cause a processing unit to perform the steps of the method as disclosed in relation to the third aspect, when said program code means are executed by the processing unit.

In some embodiments said computer program product comprises a non-transitory computer-readable medium having stored thereon the program code means.

The different aspects of the present disclosure can be implemented in different ways including image recording, endoscopic systems, a methods of recording images and computer program product described above and in the following, each yielding one or more of the benefits and advantages described in connection with at least one of the aspects described above, and each having one or more preferred embodiments corresponding to the preferred embodiments described in connection with at least one of the aspects described above and/or disclosed in the dependent claims. Furthermore, it will be appreciated that embodiments described in connection with one of the aspects described herein may equally be applied to the other aspects.

### Brief description of the drawings

The above and/or additional objects, features and advantages of the present disclosure, will be further elucidated by the following illustrative and non-limiting detailed description of embodiments of the present disclosure, with reference to the appended drawings, wherein:
Fig. 1 shows an example of an endoscope.
Fig. 2 shows an example of a display unit that can be connected to the endoscope shown in Fig. 1.
Fig. 3 shows a schematic drawing of an endoscope system according to an embodiment of the disclosure.
Fig. 4 shows a flowchart of a method 400 of recording images captured by an image capturing device of an endoscope according to an embodiment of the disclosure.
Fig. 5a-e illustrates a method of recording images captured by an image capturing device of an endoscope according to an embodiment of the disclosure.

### Detailed description

In the following description, reference is made to the accompanying figures, which show by way of illustration how the embodiments of the present disclosure may be practiced.

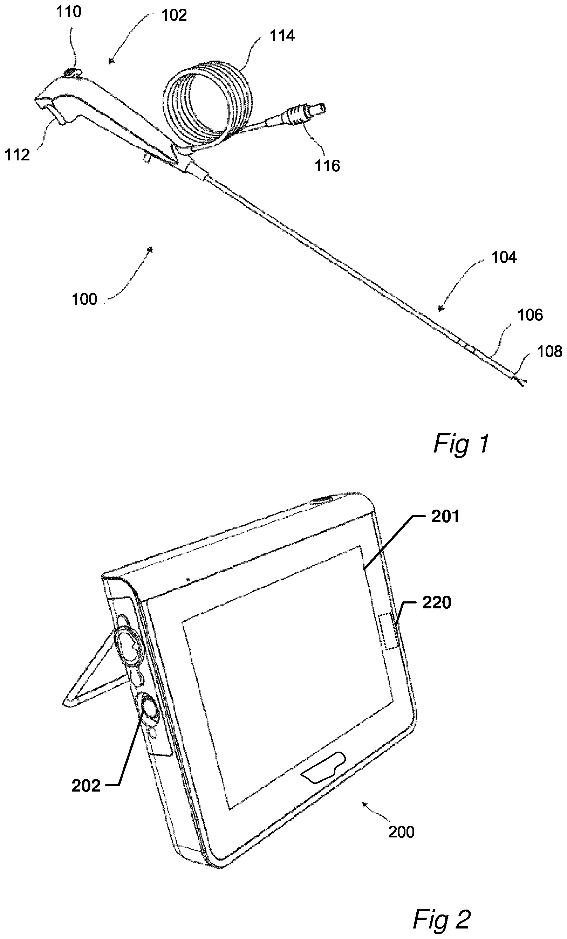

Fig. 1 illustrates an example of an endoscope 100. This endoscope may be adapted for single-use. The endoscope 100 is provided with a handle 102 attached to an insertion tube 104 provided with a bending section 106. The endoscope 100 may also be designed without a bending section 106. The illustrated endoscope 100 is a flexible endoscope with a flexible insertion tube 104, but the principle of the invention can also be used for rigid endoscopes, such as laryngoscopes or laparoscopes.

The insertion tube 104 as well as the bending section 106 may be provided with one or several working channels such that instruments, such as a gripping device or a catheter, may extend from the tip and be inserted into a human body via the endoscope. One or several exit holes of the one or several channels may be provided in a tip part 108 of the endoscope 100. In addition to the exit holes, a camera sensor, such as a CMOS sensor or any other image capturing device, as well as one or several light sources, such as light emitting diodes (LEDs), fiber, or any other light emitting devices, may be placed in the tip part 108. By having the camera sensor and the light sources and a display unit 200, illustrated in Fig. 2, configured to display images based on image data captured by the camera sensor, an operator is able to see and analyze an inside of the human body in order to for instance localize a position for taking a sample. In addition, the operator will be able to control the instrument in a precise manner due to the provided visual feedback. Further, since some diseases or health issues may result in a shift in natural colors or other visual symptoms, the operator is provided with valuable input for making a diagnosis based on the image data provided via the image capturing device and the display unit 200. The display unit comprises a display 201, a processing unit 220 (only schematically shown), and a connection port 202 for operationally connecting the endoscope to the processing unit 220. The connection port 202 may further be used to provide power to the endoscope.

In order to make it possible for the operator to direct the camera sensor such that different field of views can be achieved, the endoscope has a bending section 106 that can be bent in different directions with respect to the insertion tube 104. The bending section 106 may be controlled by the operator by using a knob 110 placed on the handle 102. The handle 102 illustrated in Fig. 1 is designed such that the knob 110 is controlled by a thumb of the operator, but other designs are also possible. In order to control a gripping device or other device provided via a working channel a push button 112 may be used. The handle 102 illustrated in Fig. 1 is designed such that a index finger of the operator is used for controlling the gripping device, but other designs are also possible.

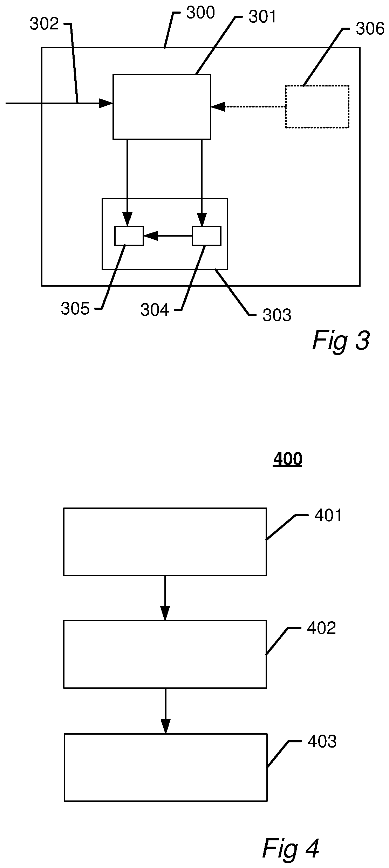

Fig. 3 show a schematic drawing of an image recording unit 300 for recording endoscope images captured by an image capturing device of an endoscope according to an embodiment of the disclosure. The image recording unit 300 comprises, a memory unit 303, and a processing unit 301. The processing unit 301 is operationally connectable 302 to the image capturing device and display. The processing unit 301 is configured to continuously receive images captured by the image capturing device of the endoscope, and continuously update a temporary image buffer 304 stored in the memory unit 303 with the received images. The temporary image buffer 304 stores a stream of images received within a first period back in time e.g. the last 30 seconds. It is also possible that the temporary image buffer 304 stores the entire stream of images received within a current endoscopic procedure, where the temporary image buffer 304 first is reset before another endoscopic procedure is performed. The processing unit 301 is further configured to in response to a received user input, store permanently a stream of images 305 captured by the image capturing device in the memory unit 303. The user input may be received from an input unit 306 of the image recording unit 300 e.g. the image recording unit 300 may comprise the display and the display may be a touch display that also function as input unit, alternatively the image recording unit 300 may be provided with one or more buttons for providing the user input. It is also possible that the user input may be received from another unit e.g. the endoscope. The permanently stored stream of images 305 includes a subset of the stream of images stored in the temporary image buffer 304 at the time when the user input was received, where the subset is selected dependent on the user input so that a user may select the point back in time where the user wants the permanently stored stream of images 305 to start. Consequently, by allowing the user to start recording video from a point back in time, the risk that the user misses an important clinical moment may be reduced. Furthermore, by allowing the user to select a particular point back in time, only the clinical important moment may be captured and not a long period of time before the clinical important moment occurred. This may reduce the memory size of the permanently stored stream of images and further make it easier to find the clinical important moment in the permanently stored stream of images.

Fig. 4 shows a flowchart of a method 400 of recording images captured by an image capturing device of an endoscope according to an embodiment of the disclosure. The method starts in step 401 with continuously receiving images captured by the image capturing device of the endoscope. Next in step 402, a temporary image buffer stored in one or more memory units is continuously updated with the received images, the temporary image buffer storing a stream of images received within a first period back in time. Finally, in step 403, in response to a received user input, a stream of images recorded by the image capturing device is stored permanently in the one or more memory units, wherein the permanently stored stream of images includes a subset of the stream of images stored in the temporary image buffer at the time when the user input was received, and wherein the subset is selected dependent on the user input so that a user may select the point back in time where the user wants the permanently stored stream of images to start.

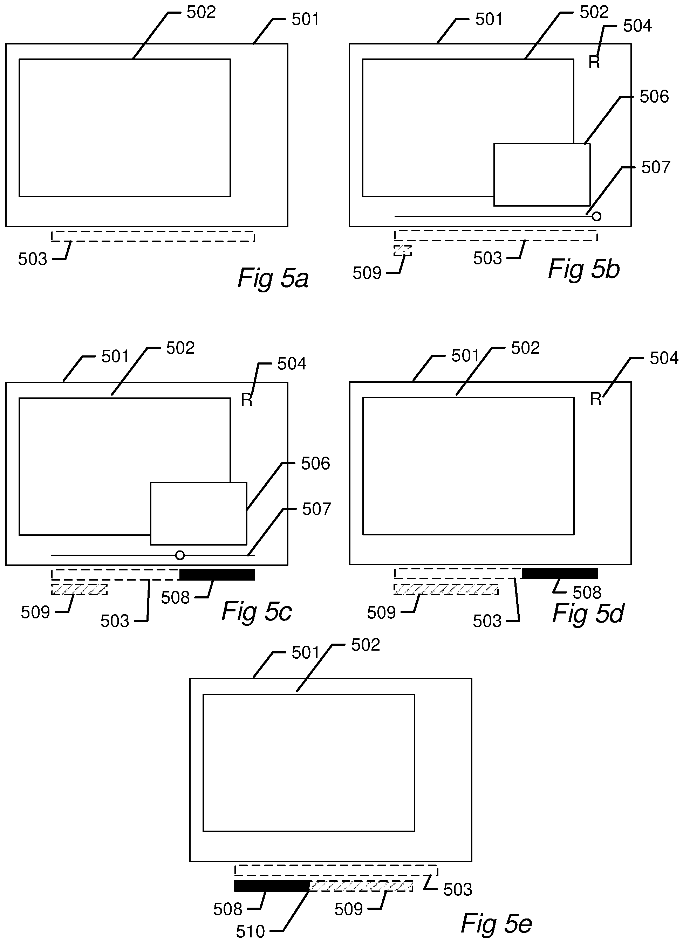

Fig. 5a-e illustrates a method of recording images captured by an image capturing device of an endoscope according to an embodiment of the disclosure. Fig. 5a-e shows a display 501 of an image recording unit at five different points in time. The image recording unit comprises a processing unit (not shown) and the display 501. The image recording unit may be identical or similar to the display unit disclosed in relation to Fig. 3. In this embodiment, the display 501 is a touch display allowing the user to provide user input to the image recording unit. Fig. 5a shows how the display looks before a user has initiated a recording of a stream of images. The processing unit is configured to control the display 501 to display live images recorded by the image capturing device of the endoscope in a first display zone 502 of the display 501. The remaining part of the display 501 may be used to show other relevant information such as patient information or parameter values of the display or the endoscope. Below the display is schematically shown a temporary image buffer 503 storing a stream of images received by the processing unit within a first period back in time. Fig. 5b shows the display after the processing unit has received a first user input. The processing unit is configured to, in response to the first user input, start permanently storing the images received from the image capturing device forming a first stream of images 509 and display images of the temporary image buffer in a second display zone 506. The processing unit may be configured to control the display to show a symbol 504 indicating that a recording is being performed. In this embodiment, the second display zone 506 partly overlaps the first display zone 502. The processing unit is configured to select a subset of the stream of images stored in the temporary image buffer 503 based on a second user input as explained in the following. The processing unit is configured to replay the stream of images stored in the temporary image buffer in the second display zone 506. The processing unit in response to the first user input is further configured to control the display to display a scrubber bar 507. Fig. 5c shows the display 501 after a third user input has been received, the third user input being received after the first user input but before the second user input. The third user input is a selection and movement along the scrubber bar, the stream of images stored in the temporary image buffer are being replayed (in reverse) in response to the selection and movement of the scrubber bar 507 in the second display zone 506, and wherein the second user input is a release of the scrubber bar 507. The selection of the subset of the stream of images stored in the temporary image buffer 503 is based on the image of the temporary image buffer shown in the second display zone 506 at the time when the second user input is received, i.e. when the scrubber bar 507 is released. The dark part 508 of the temporary image buffer 503 shows the selection of the subset. Fig. 5d shows the display 501 after the second user input has been received. The selection of the subset of the stream of images stored in the temporary image buffer 503 has been done, but the length of the first stream of images 509 is growing as the user has not stopped the recording. Fig. 5e shows the display 501 after the user has stopped the recording e.g. via a fourth user input, whereby a stream of images 510 is permanently stored comprising the subset 508 and the first stream of images 509.

Although some embodiments have been described and shown in detail, the invention is not restricted to them, but may also be embodied in other ways within the scope of the subject matter defined in the following claims. In particular, it is to be understood that other embodiments may be utilised and structural and functional modifications may be made without departing from the scope of the present invention.

In device claims enumerating several means, several of these means can be embodied by one and the same item of hardware. The mere fact that certain measures are recited in mutually different dependent claims or described in different embodiments does not indicate that a combination of these measures cannot be used to advantage.

It should be emphasized that the term "comprises/comprising" when used in this specification is taken to specify the presence of stated features, integers, steps or components but does not preclude the presence or addition of one or more other features, integers, steps, components or groups thereof.

## Claims

1. An image recording unit for recording endoscope images captured by an image capturing device of an endoscope, the image recording unit comprising a processing unit, the processing unit being operationally connectable to the image capturing device, a display, and one or more memory units, wherein the processing unit is configured to:
continuously receive images captured by the image capturing device of the endoscope;
continuously update a temporary image buffer stored in the one or more memory units with the received images, the temporary image buffer storing a stream of images received within a first period back in time;
in response to a received user input, storing permanently a stream of images recorded by the image capturing device in the one or more memory units, wherein the permanently stored stream of images includes a subset of the stream of images stored in the temporary image buffer, and wherein the subset is selected dependent on the user input so that a user may select the point back in time where the user wants the permanently stored stream of images to start.

2. An image recording unit according to claim 1, wherein the image recording unit comprises the one or more memory units.

3. An image recording unit according to claims 1 or 2, wherein the length of the first period is at least 8 second, at least 10 seconds, at least 30 seconds, or at least 1 minute, and preferably max 30 minutes.

4. An image recording unit according to any one of claims 1 to 3, wherein the user may select between at least three points back in time.

5. An image recording unit according to any one of claims 1 to 4, wherein the the processing unit is configured to control the display to continuously display live images captured by the image capturing device of the endoscope.

6. An image recording unit according to claim 5, wherein the user input comprises a first user input and a second user input, the second user input being received after the first user input, wherein the processing unit in response to the first user input is configured to start storing the images received from the image capturing device forming a first stream of images, and wherein the processing unit in response to the second user input is configured to select the subset of the stream of images stored in the temporary image buffer, and wherein the stream of images permanently stored comprises the subset and the first stream of images.

7. An image recording unit according to claim 6, wherein the processing unit in response to the first user input is configured to control the display to display live images recorded by the image capturing device of the endoscope in a first display zone the display, and further images of the temporary image buffer in a second display zone of the display.

8. An image recording unit according to claim 7, wherein the processing unit is configured to after having received the first user input replay the stream of images stored in the temporary image buffer in the second display zone preferably in a reverse direction, and wherein the processing unit further is configured to in response to the second user input select the subset of the stream of images stored in the temporary image buffer based on the image of the temporary image buffer shown in the second display zone at the time when the second user input is received.

9. An image recording unit according to claim 8, wherein the user input further comprises a third user input, the third user input being received after the first user input but before the second user input, the display is a touch display, and wherein the processing unit in response to the first user input is further configured to control the display to display a scrubber bar, and wherein the third user input is a selection and / or movement along the scrubber bar, the replay of the stream of images stored in the temporary image buffer is done in response to the selection and / or movement of the scrubber bar, and wherein the second user input is a release of the scrubber bar.

10. An image recording unit according to any one of claims 6 to 9, wherein the endoscope comprises one or more input elements, the image recording unit is operationally connectable to one or more input elements, and wherein the first user input and/or the second user input are received via the one or more input elements of the endoscope.

11. An image recording unit according to any one of claims 1 to 9, wherein the image recording unit is configured to provide power to an image capturing device of an endoscope.

12. An endoscopic system comprising an image recording unit according to any one of claims 1 to 11 and an endoscope comprising an image capturing device.

13. A method of recording images captured by an image capturing device of an endoscope, the method comprises the steps of:
continuously receive images captured by the image capturing device of the endoscope;
continuously update a temporary image buffer stored in one or more memory units with the received images, the temporary image buffer storing a stream of images received within a first period back in time;
in response to a received user input, storing permanently a stream of images recorded by the image capturing device in the one or more memory units, wherein the permanently stored stream of images includes a subset of the stream of images stored in the temporary image buffer, and wherein the subset is selected dependent on the user input so that a user may select the point back in time where the user wants the permanently stored stream of images to start.

14. A computer program product comprising program code means adapted to cause a processing unit to perform the steps of the method according to claim 13, when said program code means are executed by the processing unit.

15. A computer program product according to claim 14, wherein said computer program product comprises a non-transitory computer-readable medium having stored thereon the program code means.
